# EUROPÄISCHE PATENTSCHRIFT

(11) **EP 2 198 851 B1**
(45) Veröffentlichungstag und Bekanntmachung des Hinweises auf die Patenterteilung: **07.03.2018**
(21) Anmeldenummer: 09179558.3
(22) Anmeldetag: 17.12.2009
(51) Int. Cl.: A61K 8/41, A61K 8/81, A61K 8/86, A61K 8/892, A61K 8/895, A61K 8/92, A61Q 19/00, A61K 8/06

(54) **Verdickte O/W-Emulsionen**
Thickened O/W emulsions
Emulsions huile/eau épaissies

(30) Priorität: 17.12.2008 DE 102008062398
(43) Veröffentlichungstag der Anmeldung: 23.06.2010
(73) Patentinhaber: Henkel AG & Co. KGaA, 40589 Düsseldorf (DE)
(72) Erfinder: Stadler, Iris Marina, 47249, Duisburg (DE); Bähren, Annika, 41363, Jüchen (DE); Waldmann-Laue, Marianne, 40789, Monheim (DE)

(56) Entgegenhaltungen:
- EP-A2- 1 514 537
- WO-A2-2004/000248
- WO-A2-2008/043938
- DATABASE GNPD [Online] MINTEL; 1. Oktober 2008 (2008-10-01), Beiersdorf: "Nivea Visage Q10 Plus Advanced Wrinkle Reducer", XP002740214, Database accession no. 983333
- LOEFFLER M ET AL: "ARISTOFLEX AVC: A NEW PH STABLE POLYMER FOR GELS AND O/W EMULSIONS", COSMETIC SCIENCE CONFERENCE. PROCEEDINGS, XX, XX, 1. Januar 2001 (2001-01-01), Seiten 1-98, XP001543140,
- Nn: "Aristoflex Polymers", , 1. April 2013 (2013-04-01), Seiten 1-6, XP055191314, Gefunden im Internet: URL:http://www.essentialingredients.com/pd f/AristoflexPolymersBrochure.pdf [gefunden am 2015-05-22]

## Beschreibung

Gegenstand der Erfindung sind verdickte Öl-in-Wasser-Emulsionen, die ein spezielles Verdickersystem enthalten sowie die Verwendung des Verdickungssystems zur Verdickung wässriger Systeme.
Im Stand der Technik sind viele Verdicker und Verdickerkombinationen beschrieben. Dennoch bleibt die Verdickung wässriger Systeme, insbesondere die langzeit- und temperaturstabile Verdickung eine formulierungstechnische Herausforderung. Die Rheologie solcher verdickten Zusammensetzungen ist mit Blick auf die Effektivität der Verdickung, auf die Stabilität der Verdickungswirkung und auf das Hautgefühl (insbesondere Applizierbarkeit der Zusammensetzung und deren Klebrigkeit) noch verbesserungswürdig.
Aufgabe der vorliegenden Erfindung ist es daher, ein Verdickungssystem bereitzustellen, das sich zur Bereitstellung kosmetischer Zusammensetzungen mit angenehmem Hautgefühl eignet, wobei die Verträglichkeit, Applizierbarkeit und Klebrigkeit verbessert werden. Die vorteilhaften Eigenschaften sollten über einen langen Zeitraum und auch unter rigiden Bedingungen (z.B. lange Lagerung bei erhöhten Temperaturen) erhalten bleiben.
Die WO 2004/000248 A2 offenbart kosmetische Zusammensetzungen zur Behandlung der Haut, welche ein vernetztes Copolymer aus Vinylpyrrolidon und 2-Methyl-2[(1-oxo-2-propenyl)amino]-1-propansulfonsäure, welches teilweise oder vollständig neutralisiert ist sowie einen weiteren kosmetischen Wirkstoff, ausgewählt aus Emulgatoren, Ölen sowie vernetzten Homopolymeren der Acrylsäure, enthalten. Diese Zusammensetzungen sollen eine hohe Verträglichkeit, Lager- und Temperaturstabilität sowie einen guten sensorischen Eindruck bei der Applikation aufweisen (vgl. Zusammenfassung von D5).
Die Anmelderin hat nun überraschenderweise gefunden, dass sich wässrige Zusammensetzungen hervorragend durch ein Verdickersystem, enthaltend mindestens ein vernetztes Copolymer aus Vinylpyrrolidon und 2-Methyl-2[(1-oxo-2-propenyl)amino]-1-propansulfonsäure, das teilweise oder vollständig neutralisiert ist, mindestens ein Homopolymer von Acrylsäure, das mit Allylethern von Pentaerythritol und/oder Sucrose vernetzt ist (CTFA: Carbomer) und mindestens ein α-Hydro-ω-hydroxy-polyoxydimethylsilylen (CTFA: Dimethiconol), verdicken lassen. Die Verdickung ist effektiv und über einen langen Zeitraum auch bei hohen Temperaturen stabil. Die erzielte Verdickung verleiht den Zusammensetzungen bei der Anwendung unter anderem eine leichte Handhabbarkeit und erhöht den Verbleib des Mittels bei Applikation an vorbestimmten Orten. Insbesondere die Verdickung von Öl-in-Wasser-Emulsionen gelingt mit dem neuen Verdickungssystem hervorragend.
Ein erster Gegenstand der Erfindung ist daher eine Öl-in-Wasser-Emulsion, enthaltend - bezogen auf ihr Gewicht -
a) 40 bis 82 Gew.-% Wasser,
b) 0,95 bis 1,5 Gew.-% mindestens eines O/W-Emulgators mit einem HLB-Wert von mindestens 8, wobei das gesamte Öl-in-Wasser-Emulgatorsystem einen gewichtsmittleren (gewichtsgemittelten) HLB-Wert im Bereich von 13,5 - 15,5 aufweist,
c) mindestens ein kosmetisches Öl,
d) mindestens ein vernetztes Copolymer aus Vinylpyrrolidon und 2-Methyl-2[(1-oxo-2-propenyl)amino]-1-propansulfonsäure, das teilweise oder vollständig mit Kationen des Typs N+RR'R"R''' neutralisiert ist, bei denen R, R', R" und R''' jeweils unabhängig voneinander ausgewählt sind aus -H, -CH₃, -CH₂CH₃, -CH(CH₃)₂, -CH₂CH₂OH und -CH₂CH₂CH₂OH,
e) mindestens ein Homopolymer von Acrylsäure, das mit Allylethern von Pentaerythritol und/oder Sucrose vernetzt ist (CTFA: Carbomer),
f) 0,3 bis 1 Gew.-% mindestens eines α-Hydro-ω-hydroxy-polyoxydimethylsilylen (CTFA: Dimethiconol) .
Soweit nicht anders erwähnt, beziehen sich Mengenangaben im Rahmen der vorliegenden Anmeldung immer auf die gesamte Zusammensetzung, physikalische Größen werden bei 20°C und 1013,25 mbar gemessen.
Bevorzugte erfindungsgemäße Öl-in-Wasser-Emulsionen enthalten - bezogen auf ihr Gewicht -50 bis 80 Gew.-% Wasser.
Die erfindungsgemäßen Emulsionen enthalten bezogen auf ihr Gewicht -0,95 bis 1,5 Gew.-% mindestens eines Öl-in-Wasser-Emulgators, bevorzugt mindestens einen nichtionischen Öl-in-Wasser-Emulgator, mit einem HLB-Wert von mindestens 8, wobei das gesamte Öl-in-Wasser-Emulgatorsystem einen gewichtsmittleren (gewichtsgemittelten) HLB-Wert im Bereich von 13,5 bis 15,5, aufweist. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.
Bei der Auswahl erfindungsgemäß geeigneter Öl-in-Wasser-Emulgatoren, bevorzugt nichtionischer Öl-in-Wasser-Emulgatoren, ist es besonders bevorzugt, ein Gemisch von Öl-in-Wasser-Emulgatoren, bevorzugt nichtionischen Öl-in-Wasser-Emulgatoren, einzusetzen, um die Stabilität der erfindungsgemäßen Zusammensetzungen optimal einstellen zu können. Die einzelnen Emulgatorkomponenten liefern dabei einen Anteil zum Gesamt-HLB-Wert oder mittleren HLB-Wert des Ol-in-Wasser-Emulgatorgemisches gemäß ihrem Gewichtsanteil am Gesamtgewicht der Öl-in-Wasser-Emulgatoren. Erfindungsgemäß beträgt der gewichtsmittlere HLB-Wert des Öl-in-Wasser-Emulgatorsystems 13,5 bis 15,5. Um derartige HLB-Werte zu erzielen, werden bevorzugt Öl-in-Wasser-Emulgatoren aus den HLB-Wertbereichen 10 - 14, 14 - 16 und gegebenenfalls 15 - 17 miteinander kombiniert. Selbstverständlich können die Öl-in-Wasser-Emulgatorgemische (oder Öl-in-Wasser-Emulgatorsysteme) auch Emulgatoren, bevorzugt nichtionische Emulgatoren, mit HLB-Werten im Bereich von > 7 - 10 und 17 - 20 enthalten; derartige Emulgatorgemische können erfindungsgemäß ebenfalls bevorzugt sein. Die erfindungsgemäßen Zusammensetzungen können aber in einer anderen bevorzugten Ausführungsform auch nur einen einzigen Öl-in-Wasser-Emulgator mit einem HLB-Wert im Bereich von 11 - 17, bevorzugt 12 - 15 und besonders bevorzugt 13 - 14, enthalten.

Bevorzugte erfindungsgemäße kosmetische Zusammensetzungen sind dadurch gekennzeichnet, dass die Öl-in-Wasser-Emulgatoren b) ausgewählt sind aus ethoxylierten C₈-C₂₄-Alkanolen mit durchschnittlich 8 - 100 Mol Ethylenoxid pro Mol, ethoxylierten C₈-C₂₄-Carbonsäuren mit durchschnittlich 8 - 100 Mol Ethylenoxid pro Mol, mit durchschnittlich 20 - 100 Mol Ethylenoxid pro Mol ethoxylierten Glycerinmono- und/oder diestern von linearen gesättigten und ungesättigten C₁₂-C₃₀-Carbonsäuren, die hydroxyliert sein können, insbesondere diejenigen von Myristinsäure, Palmitinsäure, Stearinsäure, 12-Hydroxystearinsäure oder von Mischungen dieser Fettsäuren, mit durchschnittlich 20 - 100 Mol Ethylenoxid pro Mol ethoxylierten Sorbitanmonoestern von linearen gesättigten und ungesättigten C₁₂-C₃₀-Carbonsäuren, die hydroxyliert sein können, insbesondere diejenigen von Myristinsäure, Palmitinsäure, Stearinsäure, 12-Hydroxystearinsäure oder von Mischungen dieser Fettsäuren, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.
Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 8 - 100, vorzugsweise 8 - 30 Mol Ethylenoxid an 1 Mol Caprylalkohol, 2-Ethylhexylalkohol, Caprinalkohol, Laurylalkohol, Isotridecylalkohol, Tridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 8 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.
Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 8 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.
Besonders bevorzugt eingesetzt werden die C₁₂-C₁₈-Alkanole oder die C₁₂-C₁₈-Carbonsäuren mit jeweils 8 - 30 Einheiten Ethylenoxid pro Molekül sowie Mischungen dieser Substanzen, insbesondere Laureth-8, Laureth-10, Laureth-12, Laureth-20, Trideceth-8, Trideceth-9, Trideceth-10, Trideceth-12, Trideceth-20, Ceteth-10, Ceteth-12, Ceteth-20, Ceteth-30, Steareth-10, Steareth-12, Steareth-20, Steareth-30, Ceteareth-10, Ceteareth-12, Ceteareth-20, Ceteareth-30, Laureth-12 und Beheneth-20.
Bevorzugte mit durchschnittlich 20 - 100 Mol Ethylenoxid pro Mol ethoxylierte Glycerinmono- und/oder -diester von linearen gesättigten und ungesättigten C₁₂ - C₃₀-Carbonsäuren, die hydroxyliert sein können, sind ausgewählt aus PEG-20 Hydrogenated Castor Oil, PEG-40 Hydrogenated Castor Oil und PEG-60 Hydrogenated Castor Oil.
Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.
Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.
Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.
Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.
Zusammenfassend sind erfindungsgemäße Öl-in-Wasser-Emulsionen bevorzugt, die mindestens einen Öl-in-Wasser-Emulgator mit einem HLB-Wert von mindestens 8 enthalten, wobei das gesamte Öl-in-Wasser-Emulgatorsystem einen gewichtsmittleren (gewichtsgemittelten) HLB-Wert im Bereich von 11 - 17,bevorzugt 13,5 bis 15,5, aufweist.
Die erfindungsgemäßen Emulsionen enthalten weiter mindestens ein kosmetisches Öl. Diese auch als Emollientien bezeichneten Substanzen haben die Aufgabe, die Haut geschmeidig zu machen und zu glätten.
Bevorzugte erfindungsgemäße Emulsionen enthalten als Emollient mindestens ein bei 20 ° C flüssiges Öl, das keine Duftstoffkomponente und kein etherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol, Octyldodecanol und 2-Ethylhexylalkohol. Weitere bevorzugte Ölkomponenten sind Mischungen aus Guerbetalkoholen und Guerbetalkoholestern, z.B. das Handelsprodukt Cetiol® PGL (Hexyldecanol und Hexyldecyllaurat. Weitere erfindungsgemäß bevorzugte Emollient-Öle sind ausgewählt aus den Triglyceriden von linearen oder verzweigten, gesättigten oder ungesättigten, gegebenenfalls hydroxylierten C₈-₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol® 318, Myritol® 331 (Cognis) oder Miglyol® 812 (Hüls) mit unverzweigten Fettsäureresten sowie Glyceryltriisostearin und die Handelsprodukte Estol® GTEH 3609 (Uniqema) oder Myritol® GTEH (Cognis) mit verzweigten Fettsäureresten. Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat. Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether. Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat, Hexyldecyllaurat, Isodecylneopentanoat, Isononylisononanoat, 2-Ethylhexylpalmitat und 2-Ethylhexylstearat. Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat. Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether, PPG-9-Butylether, PPG-10-Butandiol und PPG-15-Stearylether. Weitere erfindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI. Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat oder die Ester der DE 19756454 A1. Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Es kann erfindungsgemäß außerordentlich bevorzugt sein, Mischungen der vorgenannten Öle einzusetzen. Weitere erfindungsgemäß bevorzugte Ölkomponenten sind ausgewählt aus Siliconölen und Kohlenwasserstoffölen. Erfindungsgemäß bevorzugte Siliconöle sind ausgewählt aus Dialkyl- und Alkylarylsiloxanen, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Weitere erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning® 200 (0, 65 cSt) und Dow Corning® 200 (1,5 cSt) von Dow Corning enthalten sind. Weitere erfindungsgemäß bevorzugte Siliconöle sind ausgewählt aus nichtflüchtigen höhermolekularen linearen Dimethylpolysiloxanen, im Handel erhältlich z. B. unter der Bezeichnung Dow Corning® 190, Dow Corning® 200 Fluid mit Viskositäten im Bereich von 5 - 100 cSt, bevorzugt 5 - 50 cSt oder auch 5 - 10 cSt, und Baysilon® 350 M. Erfindungsgemäß bevorzugte natürliche und synthetische Kohlenwasserstoffe sind ausgewählt aus Paraffinölen, Isohexadecan, Isoeicosan, (ggf. hydrierten) Polyisobutenen und Polydecenen, die beispielsweise unter der Bezeichnung Emery® 3004, 3006, 3010 oder unter der Bezeichnung Ethylflo® von Albemarle oder Nexbase® 2004G von Nestle erhältlich sind, sowie 1,3-Di-(2-ethylhexyl)-cyclohexan (Cetiol®S). Besonders bevorzugte erfindungsgemäße Emulsionen sind dadurch gekennzeichnet, dass das/die vorzugsweise bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind. In einer weiteren bevorzugten Ausführungsform der Erfindung weist ein Anteil der Ölkomponenten von mindestens 80 Gew.-% einen Brechungsindex n_{D} von 1,39 - 1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen. Neben den Ölen, das heißt, den Substanzen, die unter Normalbedingungen flüssig vorliegen, können auch bei Normalbedingungen fest vorliegende Lipid- oder Wachskomponenten als Emollient verwendet werden. Besonders bevorzugte Lipid- oder Wachskomponenten sind ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, insbesondere die Handelsprodukte Novata® (ex Cognis), besonders bevorzugt Novata® AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol® CSS), Cetylpalmitat (z. B. Cutina® CP) und Myristylmyristat (z. B. Cetiol® MM). Erfindungsgemäß bevorzugte Emollients sind weiterhin natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren. Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie den erfindungsgemäßen Hautbehandlungsmitteln ausgezeichnete sensorische Eigenschaften und eine hohe Stabilität verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben. Insbesondere können als Wachskomponente C₁₆₋₃₆-Alkylstearate und C₁₈-₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf. Eine besonders bevorzugte Ausführungsform der Erfindung enthält als Wachskomponente ein C₂₀-C₄₀-Alkylstearat. Dieser Ester ist unter den Namen Kesterwachs® K82H oder Kesterwachs® K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs® K62 bekannt und wird von Koster Keunen Inc. vertrieben. Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax® HGL-C). Erfindungsgemäß ist als Wachskomponente hydriertes Rizinusöl, erhältlich z.B. als Handelsprodukt Cutina® HR, besonders bevorzugt. Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax® AW 1C (C₁₈ - C₃₆-Fettsäuren) oder Cutina® FS 45 (Palmitin- und Stearinsäure).
Das in den erfindungsgemäßen Zusammensetzungen als Bestandteil d) der Verdickerkombination enthaltene vernetzte Copolymer aus Vinylpyrrolidon und 2-Methyl-2[(1-oxo-2-propenyl)-amino]-1-propan-sulfonsäure, das teilweise oder vollständig mit Kationen des Typs N⁺RR'R''R''' neutralisiert ist, bei denen R, R', R" und R''' jeweils unabhängig voneinander ausgewählt sind aus -H, -CH₃, -CH₂CH₃, - CH(CH₃)₂, -CH₂CH₂OH und -CH₂CH₂CH₂OH, ist erhältlich durch Copolymerisation dieser beiden Monomere in Gegenwart von Ammoniumhydroxid und einem Vernetzungsmittel.
Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.
Bevorzugte erfindungsgemäße Öl-in-Wasser-Emulsionen enthalten bezogen auf ihr Gewicht 0,05 bis 3 Gew.-%, vorzugsweise 0,1 bis 2 Gew.-%, besonders bevorzugt 0,2 bis 1,5 Gew.-%, weiter bevorzugt 0,25 bis 1,25 Gew.-% und insbesondere 0,5 bis 1 Gew.-% mindestens eines vernetzten Copolymers d) aus Vinylpyrrolidon und 2-Methyl-2[(1-oxo-2-propenyl)amino]-1-propansulfonsäure, das teilweise oder vollständig neutralisiert ist.

Vorzugsweise werden die vernetzten Copolymere d) in einem bestimmten Gewichtsverhältnis zu den Polymeren e) eingesetzt. Besonders bevorzugte erfindungsgemäße Öl-in-Wasser-Emulsionen sind dadurch gekennzeichnet, dass das Gewichtsverhältnis von d) zu e) 2:1 bis 1:2, vorzugsweise 3:2 bis 2:3, besonders bevorzugt 5:4 bis 4:5 und insbesondere 1:1 beträgt.
Die erfindungsgemäßen Emulsionen enthalten als Inhaltsstoff e) mindestens ein Homopolymer von Acrylsäure, das mit Allylethern von Pentaerythritol und/oder Sucrose vernetzt ist (CTFA: Carbomer).

Als weiteren Bestandteil f) enthalten die erfindungsgemäßen Emulsionen 0,3 bis 1 Gew.-% mindestens eines α-Hydro-ω-hydroxy-polyoxydimethylsilylen (CTFA: Dimethiconol). Dimethiconole werden auch als Dihydroxypolydimethylsiloxane oder "Dimethylsilandiol Homopolymer, Silanol-terminiert" bezeichnet. Besonders bevorzugte erfindungsgemäße Öl-in-Wasser-Emulsionen sind dadurch gekennzeichnet, dass sie - bezogen auf ihr Gewicht -0,4 bis 0,7 Gew.-% Dimethiconole enthalten.
Oft lassen sich Dimethicnole besonders gut in kosmetische Zusammensetzungen einarbeiten, wenn sie vorher mit cyclischen Silikonen (CTFA: Cyclomethicone) vermischt werden.

Erfindungsgemäß besonders geeignet ist dabei ein cyclisches Siloxan mit fünf Si-Atomen, so dass erfindungsgemäß bevorzugte Öl-in-Wasser-Emulsionen dadurch gekennzeichnet sind, dass sie zusätzlich - bezogen auf ihr Gewicht - 0,5 bis 10 Gew.-%, vorzugsweise 0,75 bis 7,5 Gew.-%, besonders bevorzugt 1,0 bis 5 Gew.-% und insbesondere 2,0 bis 3,5 Gew.-% Cyclopentasiloxan enthalten.
Erfindungsgemäß bevorzugte Emulsionen enthalten darüber hinaus mindestens ein Silikonelastomer, wobei besonders bevorzugte Öl-in-Wasser-Emulsionen zusätzlich 0,05 - 5 Gew.%, bevorzugt 0,1 - 2 Gew.% und besonders bevorzugt 0,15 - 1 Gew.%, weiter bevorzugt 0,2 bis 0,75 Gew.-% und insbesondere 0,3 bis 0,5 Gew.-%, jeweils bezogen auf die gesamte Emulsion, mindestens eines Siliconelastomers, das in einem Silicon-basierten Gel vorliegt, enthalten.
Erfindungsgemäß bevorzugte Siliconelastomere sind erhältlich durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist. Entsprechende Öl-in-Wasser-Emulsionen, bei denen das Siliconelastomere erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, sind erfindungsgemäß bevorzugt.
Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen. Entsprechende Öl-in-Wasser-Emulsionen, bei denen das Organopolysiloxan mit mindestens 2 C₂-C₁₀-Alkenyl-Gruppen mit terminaler Doppelbindung im Molekül ausgewählt ist aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen, sind erfindungsgemäß bevorzugt.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren. Erfindungsgemäß besonders bevorzugte Siliconelastomere, die durch die Vernetzung eines Organopolysiloxans erhalten werden, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist und die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040, Dow Corning 9045 und Dow Corning 9041.
Entsprechende Öl-in-Wasser-Emulsionen, bei denen das vernetzende Organopolysiloxan mit mindestens zwei Silicon-gebundenen Wasserstoffatomen ausgewählt ist aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-Siloxan-Copolymeren, sind erfindungsgemäß bevorzugt.
Weitere erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil®-Serie, insbesondere Gransil SR-CYC (Cyclomethicone and Stearyl-Vinyl/Hydromethylsiloxane Copolymer), Gransil® RPS Gel (INCI-Bezeichnung: Cyclopentasiloxane and Polysilicone-11), Gransil® GCM-4 (INCI-Bezeichnung: Cyclotetrasiloxane and Polysilicone-11), Gransil®GCM-5 (INCI-Bezeichnung: Cyclopentasiloxane and Polysilicone-11), Gransil® RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil® IDS (INCI-Bezeichnung: Isododecane (and) Cyclotetrasiloxane (and) Polysilicone-11), Gransil®PC-12 (INCI-Bezeichnung: Isododecane (and) Polysilicone-11), Gransil®IDS-5 (INCI-Bezeichnung: Isododecane (and) Cyclopentasiloxane (and) Polysilicone-11), Gransil®APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil®DMCM-5 (INCI-Bezeichnung: Dimethicone and Cyclopentasiloxane and Polysilicone-11), Gransil®DMG-6 mit Dimethicone (6 cSt) (INCI-Bezeichnung: Dimethicone and Polysilicone-11), Gransil®DMG-20 mit Dimethicone (20 cSt) (INCI-Bezeichnung: Dimethicone and Polysilicone-11), Gransil® AM-8 Gel (INCI-Bezeichnung: Caprylyl Methicone and Cyclopentasiloxane and Polysilicone-11), Gransil® DM 5 mit Dimethicone (5 cSt) (INCI-Bezeichnung: Dimethicone and Polysilicone-11), Gransil® DMID (INCI-Bezeichnung: Dimethicone and Isododecane and Polysilicone-11), Gransil® PM (INCI-Bezeichnung: Phenyl Trimethicone and Polysilicone-11), Gransil® ININ (INCI-Bezeichnung: Isononyl Isononanoate (and) Polysilicone-11).
Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil® MLB (INCI-Bezeichnung: Cyclopentasiloxane and Polysilicone-11 and Beeswax), Gransil® PS (INCI-Bezeichnung: Cyclotetrasiloxane and Polysilicone-11 and Petrolatum), Gransil® PS-5 (INCI-Bezeichnung: Cyclopentasiloxane and Polysilicone-11 and Petrolatum), Gransil® DMG-20 P mit Dimethicone (20 cSt) und Petrolatum (INCI-Bezeichnung: Dimethicone and Polysilicone-11 and Petrolatum), Gransil® RJO (INCI-Bezeichnung: Cyclopentasiloxane and Polysilicone-11 and Jojoba Oil), Gransil® LANO (INCI-Bezeichnung: Cyclopentasiloxane and Polysilicone-11 and Lanolin), Gransil® OHS-5 (INCI-Bezeichnung: Cyclopentasiloxane and Polysilicone-11 and Octyl Hydroxystearate) und Gransil® DML (INCI-Bezeichnung: Dimethicone (and) Neopentyl Glycol Diheptanoate (and) Polysilicone-11).
Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien. Entsprechende Öl-in-Wasser-Emulsionen, bei denen das Siliconelastomere durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist, sind erfindungsgemäß bevorzugt.
Insbesondere Öl-in-Wasser-Emulsionen, bei denen das Siliconelastomer ausgewählt ist aus nicht-emulgierenden Siliconelastomeren, sind erfindungsgemäß bevorzugt.
Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,15 - 0,5 Gew.-%, außerordentlich bevorzugt 0,2 - 0,3 Gew.-%, jeweils bezogen auf das Gesamtgewicht der erfindungsgemäßen Zusammensetzung, enthalten. Bevorzugte optionale Inhaltsstoffe der erfindungsgemäßen kosmetischen Mittel können über ihre Funktion definiert werden. Natürlich können manche Inhaltsstoffe auch multifunktionell sein. Bevorzugte Inhaltsstoffe der erfindungsgemäßen Mittel, vorzugsweise Kosmetikprodukte können sein:
Absorptionsmittel, antimikrobielle Stoffe, schweißhemmende Wirkstoffe, Antischaummittel, Bindemittel, Pflanzenextrakte, Bleichmittel, Chelatbildner, Deodorant-Wirkstoffe, Emulsionsstabilisatoren, Enthaarungsmittel und Wirkstoffe mit Haarwuchs inhibierender Wirkung, Emollients, Feuchtigkeitsspender, insbesondere vor wasserlösliche mehrwertige C₂ - C₉-Alkanole mit 2 - 6 Hydroxylgruppen und/oder wasserlösliche Polyethylenglycole mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fucose, Rhamnose, Fructose, Glucose, Maltose, Maltitol, Mannit, Inosit, Sucrose, Trehalose und Xylose sind erfindungsgemäß geeignet. Bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Besonders bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist; Filmbildner, wobei bevorzugte feuchtigkeitsspeichernde Filmbildner ausgewählt sind aus Polysacchariden, die mindestens einen Desoxyzucker-Baustein enthalten, besonders bevorzugt aus den Handelsprodukten Fucogel® (INCI-Bezeichnung Biosaccharide Gum-1) von Solabia, Rhamnosoft® (INCI-Bezeichnung Biosaccharide Gum-2) von Solabia, Fucogenol® (INCI-Bezeichnung Biosaccharide Gum-3) von Solabia und Glycofilm® (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus® von Solabia, Glycosaminoglycanen, besonders bevorzugt Hyaluronsäure, Dextran, Dextransulfat, Chondroitin-4-sulfat und Chondroitin-6-sulfat; weiterhin Farbstoffe, Konservierungsstoffe, hautkühlende Wirkstoffe, wie z. B. Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2-Hydroxymethyl-3,5,5-trimethylcyclohexanol sowie Mischungen dieser Substanzen, pH-Wert-Regler/ Puffersubstanzen, Tenside/waschaktive Substanzen, insbesondere anionische Tenside, insbesondere in Mengen von 0,1 bis 5 Gew.-%, nichtionische Tenside, insbesondere in Mengen von 0,1 bis 5 Gew.-%, kationische Tenside, insbesondere in Mengen von 0,1 bis 2 Gew.-%, amphotere Tenside, insbesondere in Mengen von 0,1 bis 5 Gew.-%, Treibgase, Trübungsmittel und UV-Absorber/Lichtfiltersubstanzen.
Vorgenannte Inhaltsstoffe können gemäß weiterer bevorzugter Ausführungsformen als einziger Zusatz oder in beliebigen Kombinationen in den erfindungsgemäßen Mitteln enthalten sein.
Nach einer anderen bevorzugten Ausführungsform kann das erfindungsgemäße Mittel frei von Aniontensid sein. Nach einer anderen bevorzugten Ausführungsform kann das erfindungsgemäße Mittel frei von Niotensid sein. Zur Vermeidung möglicher Inkompatibilitäten der kationischen Tenside mit in dem erfindungsgemäßen Mittel enthaltenen anionischen Tensiden werden möglichst aniontensidverträgliches und/oder ggf. möglichst wenig kationisches Tensid eingesetzt oder in einer besonderen Ausführungsform der Erfindung gänzlich auf kationische Tenside verzichtet.
Nach einer anderen bevorzugten Ausführungsform kann das erfindungsgemäße Mittel frei von amphoteren Tensiden sein.
Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.
Es sei an dieser Stelle darauf hingewiesen, dass sich die Angabe Gew.-%, sofern es nicht anders angegeben ist, jeweils auf das gesamte Mittel bezieht.
Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.-%, in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel.
Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.
Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.
Die genannten Ober- und Untergrenzen können natürlich sinnvoll kombiniert werden, z.B. zu Wassergehalten von 60-80 Gew.-% oder 10-30 Gew.-% usw.
Weitere bevorzugte erfindungsgemäße Zusammensetzungen sind dadurch gekennzeichnet, dass zur Verbesserung der Hautfeuchtigkeit unter Verzicht auf komedogene Substanzen mindestens ein alkyl- oder hydroxyalkylsubstituierten Harnstoff der allgemeinen Formel (UREA-1) enthalten ist, 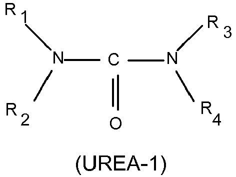 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.
Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Bis-N,N'- (2-hydroxyethyl)harnstoff. Erfindungsgemäß besonders bevorzugte Zusammensetzungen sind dadurch gekennzeichnet, dass sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance von AkzoNobel (vormals National Starch) als ca. 45 - 55 %ige wässrige Lösung mit einem Gehalt an Harnstoff und Ammoniumlactat erhältlich.
In einer bevorzugten Ausführungsform enthalten die erfindungsgemäßen Zusammensetzungen mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe.
Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*)*,* der die Synthese von Collagen in dermalen Fibroblasten induziert, freizusetzen. Derartige Aminosäureoligomere können als Wirkstoffe gegen die Hautalterung verwendet werden.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN®-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produkts Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt. ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.
Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C® von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine® von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind. Proteinhydrolysate können naturgemäß auch monomere Aminosäuren und Oligopeptide enthalten; ihre Zusammensetzung ist normalerweise nicht definiert.
Ebenfalls bevorzugt ist der Einsatz von Acylderivaten der Proteinhydrolysate, z. B. in Form ihrer Fettsäure-Kondensationsprodukte. Ein weiterer erfindungsgemäß bevorzugter Wirkstoff ist das Peptidderivat L-Glutamylaminoethyl-indol (erhältlich z. B. unter dem Handelsnamen Glistin von Exsymol).
Erfindungsgemäß bevorzugt sind auch kationisierte Proteinhydrolysate.
In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, und E und den Estern der vorgenannten Substanzen. Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.
Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt. 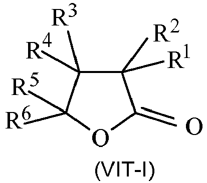
Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.
In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton.

Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.
In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.
Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel® von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft® von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol® von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm® von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus® von Solabia.
Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Ein weiterer Gegenstand der vorliegenden Erfindung ist die Verwendung von Mischungen aus
a) mindestens einem vernetzten Copolymer aus Vinylpyrrolidon und 2-Methyl-2[(1-oxo-2-propenyl)amino]-1-propansulfonsäure, das teilweise oder vollständig neutralisiert ist,
b) mindestens einem Homopolymer von Acrylsäure, das mit Allylethern von Pentaerythritol und/oder Sucrose vernetzt ist (CTFA: Carbomer),
c) mindestens einem α-Hydro-ω-hydroxy-polyoxydimethylsilylen (CTFA: Dimethiconol)
zur Verdickung von Öl-in-Wasser-Emulsionen.
Weiter bevorzugte Verwendungen sind dadurch gekennzeichnet, dass die Mischung zusätzlich mindestens ein Siliconelastomer, das in einem Silicon-basierten Gel vorliegt, enthält.
Bezüglich weiterer bevorzugter Ausführungsforen der erfindungsgemäßen Verwendungen gilt mutatis mutandis das zu den erfindungsgemäßen Mitteln Gesagte.

### Beispiele:

Es wurden folgende Emulsionen hergestellt (Angaben in Gew.-%):

| | **1** | **2** | **3** | **4** | **5** |
|---|---|---|---|---|---|
| PEG-40 HYDROGENATED CASTOR OIL | 0,5 | 0,4 | 1,0 | 0,45 | 0,5 |
| TRIDECETH-9 | 0,5 | 1,0 | 0,4 | 0,45 | 0,5 |
| CAPRYLIC/CAPRIC TRIGLYCERIDE | 3,0 | 2,5 | 2,0 | 5,0 | 3,75 |
| Aristoflex® AVC | 0,75 | 1,0 | 0,5 | 1,5 | 2,0 |
| Carbopol® Ultrez 10 | 0,55 | 0,75 | 0,5 | 0,8 | 0,6 |
| Dimethiconol | 0,45 | 0,5 | 0,6 | 0,75 | 1,0 |
| Cyclopentasiloxan | 2,5 | 3,0 | 2,0 | 2,2 | 2,7 |
| Dow Corning 9040 | 3,0 | 2,5 | 3,5 | 3,0 | 3,0 |
| Phenonip ME | 1,0 | 1,0 | 1,0 | 1,0 | 1,0 |
| Phytokine | 2,0 | 2,0 | 1,5 | 1,5 | 2,0 |
| Matrixyl 3000 | 3,0 | 3,0 | 2,5 | 3,0 | 2,5 |
| DSH-CN | 5,0 | 4,0 | 2,5 | 4,0 | 5,0 |
| Wasser | ad 100 | ad 100 | ad 100 | ad 100 | ad 100 |

| | | | | | |
|---|---|---|---|---|---|
| Aristoflex® AVC Ammonium-Acryloyldimethyltaurat-Vinylpyrrolidon-Copolymer (Clariant) Carbopol® Ultrez 10 Polyacrylat (INCI-Bezeichnung: Carbomer) (Lubrizol) Dow Corning 9040 Cyclomethicone/ Dimethicone Crosspolymer Phenonip ME Phenoxyethanol, Methylparaben, Ethylparaben, Propylparaben, Butylparaben, ca. 28 % Aktivsubstanz (Nipa) Phytokine HYDROLYZED SOY PROTEIN (Coletica) Matrixyl 3000 Glycerin, Aqua, Butylene Glycol, Carbomer, Polysorbate 20, Palmitoyl Oligopeptide, Palmitoyl Tetrapeptide-1 (Sederma) DSH-CN Water, Dimethylsilanol Hyaluronate (Exsymol) | | | | | |

Die Emulsionen wiesen ein angenehmes Hautgefühl auf, waren leicht applizierbar, nicht klebrig und behielten diese Eigenschaften ohne Brechen der Emulsion auch bei sechswöchiger Lagerung bei 60°C.

## Patentansprüche

1. Öl-in-Wasser-Emulsion, enthaltend - bezogen auf ihr Gewicht -
a) 40 bis 82 Gew.-% Wasser,
b) 0,95 bis 1,5 Gew.-% mindestens eines O/W-Emulgators mit einem HLB-Wert von mindestens 8, wobei das gesamte Öl-in-Wasser-Emulgatorsystem einen gewichtsmittleren (gewichtsgemittelten) HLB-Wert im Bereich von 13,5 - 15,5 aufweist,
c) mindestens ein kosmetisches Öl,
d) mindestens ein vernetztes Copolymer aus Vinylpyrrolidon und 2-Methyl-2[(1-oxo-2-propenyl)amino]-1-propansulfonsäure, das teilweise oder vollständig mit Kationen des Typs N⁺RR'R"R"' neutralisiert ist, bei denen R, R', R" und R''' jeweils unabhängig voneinander ausgewählt sind aus -H, -CH₃, -CH₂CH₃, -CH(CH₃)₂, -CH₂CH₂OH und -CH₂CH₂CH₂OH,
e) mindestens ein Homopolymer von Acrylsäure, das mit Allylethern von Pentaerythritol und/oder Sucrose vernetzt ist (CTFA: Carbomer),
f) 0,3 bis 1 Gew.-% mindestens eines α-Hydro-ω-hydroxy-polyoxydimethylsilylen (CTFA: Dimethiconol) .

2. Öl-in-Wasser-Emulsion nach Anspruch 1, **dadurch gekennzeichnet, dass** sie - bezogen auf ihr Gewicht -50 bis 80 Gew.-% Wasser enthält.

3. Öl-in-Wasser-Emulsion nach einem der Ansprüche 1 oder 2, **dadurch gekennzeichnet, dass** das Gewichtsverhältnis von d) zu e) 2:1 bis 1:2, vorzugsweise 3:2 bis 2:3, besonders bevorzugt 5:4 bis 4:5 und insbesondere 1:1 beträgt.

4. Öl-in-Wasser-Emulsion nach einem der Ansprüche 1 bis 3, **dadurch gekennzeichnet, dass** sie bezogen auf ihr Gewicht 0,05 bis 3 Gew.-%, vorzugsweise 0,1 bis 2 Gew.-%, besonders bevorzugt 0,2 bis 1,5 Gew.-%, weiter bevorzugt 0,25 bis 1,25 Gew.-% und insbesondere 0,5 bis 1 Gew.-% mindestens eines vernetzten Copolymers d) aus Vinylpyrrolidon und 2-Methyl-2[(1-oxo-2-propenyl)amino]-1-propansulfonsäure, das teilweise oder vollständig neutralisiert ist, enthält.

5. Öl-in-Wasser-Emulsion nach einem der Ansprüche 1 bis 4, **dadurch gekennzeichnet, dass** sie zusätzlich 0,05 - 5 Gew.%, bevorzugt 0,1 - 2 Gew.% und besonders bevorzugt 0,15 - 1 Gew.%, weiter bevorzugt 0,2 bis 0,75 Gew.-% und insbesondere 0,3 bis 0,5 Gew.-%, jeweils bezogen auf die gesamte Emulsion, mindestens eines Siliconelastomers, das in einem Silicon-basierten Gel vorliegt, enthält.

6. Öl-in-Wasser-Emulsion nach Anspruch 5, **dadurch gekennzeichnet, dass** das Siliconelastomere erhältlich ist durch die Vernetzung eines Organopolysiloxans, das mindestens 2 C₂-C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

7. Öl-in-Wasser-Emulsion nach einem der Ansprüche 5 oder 6, **dadurch gekennzeichnet, dass** das Siliconelastomere durch die Vernetzung eines Organopolysiloxans, das mindestens 2 C₂-C₁₀-Alkenylgruppen mit terminaler Doppelbindung in jedem Molekül enthält, mit mindestens einem alpha, omega-Dien erhältlich ist.

8. Öl-in-Wasser-Emulsion nach einem der Ansprüche 5 bis 7, **dadurch gekennzeichnet, dass** das Siliconelastomer ausgewählt ist aus nicht-emulgierenden Siliconelastomeren.

9. Öl-in-Wasser-Emulsion nach einem der Ansprüche 5 bis 8, **dadurch gekennzeichnet, dass** das Organopolysiloxan mit mindestens 2 C₂ - C₁₀-Alkenyl-Gruppen mit terminaler Doppelbindung im Molekül ausgewählt ist aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

10. Öl-in-Wasser-Emulsion nach einem der Ansprüche 5 bis 9, **dadurch gekennzeichnet, dass** das vernetzende Organopolysiloxan mit mindestens zwei Silicon-gebundenen Wasserstoffatomen ausgewählt ist aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-Siloxan-Copolymeren.

11. Öl-in-Wasser-Emulsion nach einem der Ansprüche 1 bis 10, **dadurch gekennzeichnet, dass** sie - bezogen auf ihr Gewicht - 0,4 bis 0,7 Gew.-% Dimethiconole enthält.

12. Verwendung von Mischungen aus
a) mindestens einem vernetzten Copolymer aus Vinylpyrrolidon und 2-Methyl-2[(1-oxo-2-propenyl)amino]-1-propansulfonsäure, das teilweise oder vollständig neutralisiert ist,
b) mindestens einem Homopolymer von Acrylsäure, das mit Allylethern von Pentaerythritol und/oder Sucrose vernetzt ist (CTFA: Carbomer),
c) mindestens einem α-Hydro-ω-hydroxy-polyoxydimethylsilylen (CTFA: Dimethiconol) zur Verdickung von Öl-in-Wasser-Emulsionen.

## Claims

1. An oil-in-water emulsion containing, based on its weight,
a) from 40 to 82 wt.% of water,
b) from 0.95 to 1.5 wt.% of at least one oil-in-water emulsifier having an HLB value of at least 8, wherein the overall oil-in-water emulsifier system has a weight average HLB value in the range of from 13.5 to 15.5,
c) at least one cosmetic oil,
d) at least one crosslinked copolymer of vinylpyrrolidone and 2-methyl-2[(1-oxo-2-propenyl)amino]-1-propane sulfonic acid which is partially or completely neutralized with cations of the N⁺RR'R"R"' type, in which R, R', R" and R''' are each selected, independently of one another, from -H, -CH₃, -CH₂CH₃, -CH(CH₃)₂, -CH₂CH₂OH and -CH₂CH₂CH₂OH,
e) at least one homopolymer of acrylic acid which is crosslinked with allyl ethers of pentaerythritol and/or sucrose (CTFA: carbomer),
f) from 0.3 to 1 wt.% of at least one α-hydro-ω-hydroxy-polyoxydimethylsilylene (CTFA: dimethiconol).

2. The oil-in-water emulsion according to claim 1, **characterized in that** it contains, based on its weight, from 50 to 80 wt.% of water.

3. The oil-in-water emulsion according to one of claims 1 or 2, **characterized in that** the weight ratio of d) to e) is from 2:1 to 1:2, preferably from 3:2 to 2:3, particularly preferably from 5:4 to 4:5, and is in particular 1:1.

4. The oil-in-water emulsion according to one of claims 1 to 3, **characterized in that** it contains, based on its weight, from 0.05 to 3 wt.%, preferably from 0.1 to 2 wt.%, particularly preferably from 0.2 to 1.5 wt.%, more preferably from 0.25 to 1.25 wt.%, and in particular from 0.5 to 1 wt.%, of at least one crosslinked copolymer d) of vinylpyrrolidone and 2-methyl-2[(1-oxo-2-propenyl)amino]-1-propane sulfonic acid which is partially or completely neutralized.

5. The oil-in-water emulsion according to one of claims 1 to 4, **characterized in that** it additionally contains, based on the entire emulsion in each case, from 0.05 to 5 wt.%, preferably from 0.1 to 2 wt.%, particularly preferably from 0.15 to 1 wt.%, more preferably from 0.2 to 0.75 wt.%, and in particular from 0.3 to 0.5 wt.%, of at least one silicone elastomer present in a silicone-based gel.

6. The oil-in-water emulsion according to claim 5, **characterized in that** the silicone elastomer can be obtained by crosslinking an organopolysiloxane that contains at least two C2-C10 alkenyl groups having a terminal double bond in each molecule, with an organopolysiloxane that comprises at least two silicone-bound hydrogen atoms in each molecule.

7. The oil-in-water emulsion according to one of claims 5 or 6, **characterized in that** the silicone elastomer can be obtained by crosslinking an organopolysiloxane that contains at least two C2-C10 alkenyl groups having a terminal double bond in each molecule, with at least one alpha, omega-diene.

8. The oil-in-water emulsion according to one of claims 5 to 7, **characterized in that** the silicone elastomer is selected from non-emulsifying silicone elastomers.

9. The oil-in-water emulsion according to one of claims 5 to 8, **characterized in that** the organopolysiloxane having at least two C2-C10 alkenyl groups having a terminal double bond in the molecule is selected from methylvinylsiloxanes, methylvinylsiloxane-dimethylsiloxane copolymers, dimethylpolysiloxanes having dimethylvinylsiloxy terminal groups, dimethylsiloxane-methyl-phenylsiloxane copolymers having dimethylvinylsiloxy terminal groups, dimethylsiloxane-diphenylsiloxane-methylvinylsiloxane copolymers having dimethylvinylsiloxy terminal groups, dimethylsiloxane-methylvinylsiloxane copolymers having trimethylsiloxy terminal groups, dimethyl-siloxane-methylphenylsiloxane-methylvinylsiloxane copolymers having trimethylsiloxy terminal groups, methyl-(3,3,3-trifluoropropyl)-polysiloxanes having dimethylvinylsiloxy terminal groups, and dimethylsil-oxan-methyl-(3,3,3-trifluoropropyl)-siloxane copolymers having dimethylvinylsiloxy terminal groups.

10. The oil-in-water emulsion according to one of claims 5 to 9, **characterized in that** the crosslinked organopolysiloxane having at least two silicone-bound hydrogen atoms is selected from methylhydrogenpolysiloxanes having trimethylsiloxy terminal groups, dimethylsiloxane-methylhydrogensiloxane copolymers having trimethylsiloxy terminal groups and cyclic dimethylsiloxane-methylhydrogen-siloxane copolymers.

11. The oil-in-water emulsion according to one of claims 1 to 10, **characterized in that** it contains, based on its weight, from 0.4 wt.% to 0.7 wt.% of dimethiconol.

12. The use of mixtures of
a) at least one crosslinked copolymer of vinylpyrrolidone and 2-methyl-2[(1-oxo-2-propenyl)amino]-1-propane sulfonic acid which is partially or completely neutralized,
b) at least one homopolymer of acrylic acid which is crosslinked with allyl ethers of pentaerythritol and/or sucrose (CTFA: carbomer),
c) at least one α-hydro-ω-hydroxy-polyoxydimethylsilylene (CTFA: dimethiconol), for thickening oil-in-water emulsions.

## Revendications

1. Émulsion huile-dans-eau, contenant - par rapport à son poids -
a) 40 à 82 % en poids d'eau,
b) 0,95 à 1,5 % en poids d'au moins un émulsifiant huile-dans-eau ayant une valeur HLB d'au moins 8, tout le système d'émulsifiant huile-dans-eau ayant une valeur HLB moyenne en poids dans la gamme allant de 13,5 à 15,5,
c) au moins une huile cosmétique,
d) au moins un copolymère réticulé constitué de vinylpyrrolidone et d'acide 2-méthyl-2-[(1-oxo-2-propényl)amino]-1-propanesulfonique qui est partiellement ou totalement neutralisé par des cations du type N⁺RR'R"R''', où R, R', R" et R''' sont choisis chacun indépendamment les uns des autres parmi -H, -CH₃, -CH₂CH₃, -CH(CH₃)₂, -CH₂CH₂OH et -CH₂CH₂CH₂OH,
e) au moins un homopolymère d'acide acrylique réticulé par des éthers allyliques de pentaérythrol et/ou de saccharose (CTFA : carbomère),
f) 0,3 à 1 % en poids d'au moins un α-hydro-ω-hydroxy-polyoxydiméthylsilylène (CTFA : diméthiconol).

2. Émulsion huile-dans-eau selon la revendication 1, **caractérisée en ce qu'**elle contient - par rapport à son poids - 50 à 80 % en poids d'eau.

3. Émulsion huile-dans-eau selon l'une des revendications 1 ou 2, **caractérisée en ce que** le rapport en poids de d) à e) est de 2:1 à 1:2, de préférence de 3:2 à 2:3, de façon particulièrement préférée de 5:4 à 4:5 et en particulier de 1:1.

4. Émulsion huile-dans-eau selon l'une des revendications 1 à 3, **caractérisée en ce qu'**elle contient, par rapport à son poids, 0,05 à 3 % en poids, de préférence 0,1 à 2 % en poids, de façon particulièrement préférée de 0,2 à 1,5 % en poids, plus préférablement 0,25 à 1,25 % en poids et en particulier 0,5 à 1 % en poids d'au moins un copolymère réticulé d) constitué de vinylpyrrolidone et d'acide 2-méthyl-2[(1-oxo-2-propényl)amino]-1-propanesulfonique partiellement ou totalement neutralisé.

5. Émulsion huile-dans-eau selon l'une des revendications 1 à 4, **caractérisée en ce qu'**elle contient en plus 0,05 - 5 % en poids, de préférence 0,1 à 2 % en poids et de façon particulièrement préférée 0,15 à1 % en poids, plus préférablement 0,2 à 0,75 % en poids et en particulier 0,3 à 0,5 % en poids, à chaque fois sur la base de toute l'émulsion, d'au moins un silicone élastomère présent dans un gel à base de silicone.

6. Émulsion huile-dans-eau selon la revendication 5, **caractérisée en ce que** le silicone élastomère s'obtient est obtenu par réticulation d'un organopolysiloxane qui contient au moins deux groupes alcényle en C2 à C10 à double liaison terminale dans chaque molécule, avec un organopolysiloxane qui comporte au moins deux atomes d'hydrogène liés au silicone dans chaque molécule.

7. Émulsion huile-dans-eau selon l'une des revendications 5 ou 6, **caractérisée en ce que** le silicone élastomère s'obtient est obtenu par réticulation d'un organopolysiloxane qui contient au moins deux groupes alcényle en C2 à C10 à double liaison terminale dans chaque molécule, avec au moins un α-w-diène.

8. Émulsion huile-dans-eau selon l'une des revendications 5 à 7, **caractérisée en ce que** le silicone élastomère est choisi parmi des silicones élastomères non-émulsionnants.

9. Émulsion huile-dans-eau selon l'une des revendications 5 à 8, **caractérisée en ce que** l'organopolysiloxane comportant au moins deux groupes alcényle en C2 à C10 à double liaison terminale dans la molécule est choisi parmi les méthylvinylsiloxanes, les copolymères méthylvinylsiloxane-diméthylsiloxane, les diméthylpolysiloxanes à groupes terminaux diméthylvinylsiloxy, les copolymères diméthylsiloxane-méthylphénylsiloxane à groupes terminaux diméthylvinylsiloxy, les copolymères diméthylsiloxane-diphénylsiloxane-méthylvinylsiloxane à groupes terminaux diméthylvinylsiloxy, les copolymères diméthylsiloxane-méthylvinylsiloxane à groupes terminaux triméthylsiloxy, les copolymères diméthylsiloxane-méthylphénylsiloxane-méthylvinylsiloxane à groupes terminaux triméthylsiloxy, les copolymères méthyl-(3,3,3-trifluoropropyl)-polysiloxanes à groupes terminaux diméthylvinylsiloxy et les copolymères diméthylsiloxane-méthyl-(3,3,3-trifluoropropyl)-siloxane à groupes terminaux diméthylvinylsiloxy.

10. Émulsion huile-dans-eau selon l'une des revendications 5 à 9, **caractérisée en ce que** l'organopolysiloxane réticulé comportant au moins deux atomes d'hydrogène liés au silicone est choisi parmi les méthylhydrogénopolysiloxanes à groupes terminaux triméthylsiloxy, les copolymères diméthylsiloxane-méthylhydrogénosiloxane à groupes terminaux triméthylsiloxy et les copolymères diméthylsiloxane -méthylhydrogénosiloxane cycliques.

11. Émulsion huile-dans-eau selon l'une des revendications 1 à 10, **caractérisée en ce qu'**elle contient - par rapport à son poids - 0,4 à 0,7 % en poids de diméthiconole.

12. Utilisation de mélanges composés :
a) d'au moins un copolymère réticulé constitué de vinylpyrrolidone et d'acide 2-méthyl-2[(1-oxo-2-propényl)amino]-1-propanesulfonique partiellement ou totalement neutralisé,
b) d'au moins un homopolymère d'acide acrylique réticulé par des éthers allyliques de pentaérythrol et/ou de saccharose (CTFA : carbomère),
c) d'au moins un α-hydro-ω-hydroxy-polyoxydiméthylsilylène (CTFA : diméthiconol), pour épaissir les émulsions huile-dans-eau.
